(19) **Europäisches Patentamt / European Patent Office / Office européen des brevets**

(11) **EP 3 851 091 A1**

(12) ## EUROPEAN PATENT APPLICATION
published in accordance with Art. 153(4) EPC

(43) Date of publication:
**21.07.2021 Bulletin 2021/29**

(21) Application number: **19860936.4**

(22) Date of filing: **03.09.2019**

(51) Int Cl.:
*A61K 8/20* (2006.01)  *A61K 8/02* (2006.01)
*A61K 9/06* (2006.01)  *A61K 9/08* (2006.01)
*A61K 33/20* (2006.01)  *A61P 1/02* (2006.01)
*A61Q 11/00* (2006.01)

(86) International application number:
**PCT/JP2019/034516**

(87) International publication number:
**WO 2020/054494 (19.03.2020 Gazette 2020/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **13.09.2018 JP 2018171880**

(71) Applicant: **Tokuyama Dental Corporation
Tokyo 110-0016 (JP)**

(72) Inventors:
• **OYA, Naoyuki
  Tokyo 110-0016 (JP)**
• **KAZAMA, Hideki
  Tokyo 110-0016 (JP)**

• **HIRATA, Kouichirou
  Tokyo 110-0016 (JP)**
• **YAMAGAWA, Jyunichirou
  Tokyo 110-0016 (JP)**
• **SHIMIZU, Tomonao
  Tokyo 110-0016 (JP)**
• **KIRA, Ryuuta
  Tokyo 110-0016 (JP)**
• **TAKAHASHI, Ayumu
  Tokyo 110-0016 (JP)**
• **SAKATA, Eibu
  Tokyo 110-0016 (JP)**

(74) Representative: **Gulde & Partner
Patent- und Rechtsanwaltskanzlei mbB
Wallstraße 58/59
10179 Berlin (DE)**

(54) **TOOTH CLEANING COMPOSITION, TOOTH CLEANING AGENT, DENTAL CALCULUS REMOVAL ACCELERATOR, AND TOOTH CLEANING METHOD**

(57)    The present invention provides a tooth cleaning composition containing a weakly acidic hypochlorite solution that has an effective chloride concentration of 100-2000 (mass ppm) and a pH of 3.0-6.5.

**EP 3 851 091 A1**

## Description

### Technical Field

The present invention relates to an agent for removal of dental calculi or dental plaque (plaque), or a tooth cleaning composition effective as a removal accelerator.

### Background Art

It is well known that dental plaque (plaque) or dental calculi cause a periodontal disease. From the viewpoint of the prevention of periodontal disease, it is desirable to keep the oral cavity clean on a daily basis so that such dental plaque or dental calculi are not generated. However, since plaque has strong adhesiveness, it remains on a tooth surface when brushing is insufficient. In particular, in a portion not well cleaned such as the boundary between a tooth and gingiva (gingival sulcus), dental plaque accumulates and calcifies to form dental calculi. For this reason, periodic removal of dental calculi is recommended as part of oral care.

Dental calculi contain bacterial cells, proteins, saccharides, and the like as organic components, and continue to release a toxin that promotes periodontal disease. Most of dental calculi are formed of an inorganic salt such as hydroxyapatite ($Ca_{10}(PO_4)_6(OH)_2$) and is firmly and strongly fixed onto a tooth surface. Therefore, the removal of dental calculi is not easy. In general, as the method for removal of dental calculi, a method of physically removing dental calculi by using a dental tool called a scaler is adopted. However, due to the reasons described above, such a dental treatment takes a lot of work and time, and puts a burden not only on a dental hygienist or a dentist who performs the treatment, but also on a person to be treated (patient).

In view of such a circumstance, a dental calculus removal accelerator for facilitating the removal of dental calculi has been proposed. For example, Patent Literature 1 discloses that "a composition for softening or dissolving dental calculi, including calcium ions, orthophosphate ions, fluoride ions, and a substance having a buffering capacity in an acidic region, and being characterized in that the composition has a pH of 3 to 6.5, and further is unsaturated with respect to any one or more of hydroxyapatite, calcium quaternary phosphate, calcium tertiary phosphate, and calcium secondary phosphate, and is supersaturated with respect to both of fluoroapatite and calcium fluoride". Further, when a human tooth to which dental calculi have adhered is immersed in the composition kept at 37°C overnight, the state of action of softening or dissolving dental calculi is considered to be favorable in evaluation of scratching with a dental scaler.

In addition, Patent Literature 2 discloses that "an agent for removal of dental calculi and/or dental caries portions, including alkaline water obtained by electrolyzing water containing metal ions and/or an electrolyte". Further, Patent Literature 2 discloses that when dental calculi are immersed at 37°C for 30 minutes in alkaline water obtained by electrolyzing an aqueous solution in which sodium chloride has been dissolved in Mt. Fuji groundwater containing a minute amount of vanadium, floating dental calculi have been observed (see Example 1), and moreover, when dental calculi are brushed for 1 minute with a toothbrush soaked in a solution in which phytic acid and sodium chloride have been dissolved in alkaline water obtained similarly so as to have concentrations of 12.5% and 10%, respectively, a weight reduction of around 20% has been observed (see Example 9).

Further, Patent Literature 3 discloses "an agent for dissolving dental calculi, which contains lactic acid as an active component", and also discloses as an example, that a weight reduction of 1.0 mg has been recognized by immersing 2.0 mg of dental calculi in 1 ml of a 10% aqueous solution of lactic acid (adjusted the pH to 3.1 with a 0.1 N aqueous solution of sodium hydroxide) for 14 minutes.

In addition, Non Patent Literature 1 discloses that as the solution that facilitates (or accelerates) scaling and root planing (SRP), a solution at pH 11, which contains a hypochlorite and three amino acids consisting of leucine, glutamic acid, and lysine as active components and further contains sodium chloride, carboxymethyl cellulose, titanium dioxide, water, and sodium hydroxide, is commercially available, and also discloses evaluation results by an in vitro pilot study on this, that is, evaluation results in which a remarkable reduction effect has not been observed in the treatment time and the number of strokes required for the treatment.

### Citation List

#### Patent Literature

Patent Literature 1: JP H09-143043 A
Patent Literature 2: JP 4588348 B2
Patent Literature 3: JP 2007-126409 A

## Non Patent Literature

Non Patent Literature 1: M. Becker et al., International Journal of Dental Hygiene 16, 2018, 151-156

## Summary of Invention

### Technical Problem

As described above, there are several known so-called dental calculus removal accelerators, but most of the dental calculus removal accelerators are used with the immersion of dental calculi or a tooth or the like to which dental calculi have adhered, in the dental calculus removal accelerators for a long time. It is considered that such dental calculus removal accelerators are effective for an item that can be taken out of the oral cavity for treatment, such as a denture, but the usefulness for a treatment in a short time at a dental clinic or the like remains questionable. In this respect, as disclosed in Non Patent Literature 1, it can be admitted also from the fact that the effect may not be recognized even by a commercially available dental calculus removal accelerator (which is considered that the effect has been confirmed in a test by a provider), in view of shortening the time and reducing the number of strokes required for the treatment of SRP.

In addition, as described previously, Patent Literature 2 discloses that in a case where large amounts of phytic acid and sodium chloride are dissolved and used, brushing for around 1 minute exerts a certain effect, but the dental calculi have not yet been completely removed. Further, since phytic acid is a relatively expensive substance, it is considered that there is still room for improvement in cost.

In view of this, it is an object of the present invention to provide a tooth cleaning composition that makes the removal of dental calculi easier by a treatment in a short time, and makes the actual feeling of workload reduced, and further that does not require to use of any expensive or special reagent, in the work of the removal of dental calculi by using a scaler (probe).

### Solution to Problem

The present inventors have conducted intensive studies in order to solve the problems described above. As a result of which the present inventors have found that the above object can be achieved by using a so-called weakly acidic hypochlorite solution containing hypochlorous acid in a molecular state, and thus have completed the present invention.

That is, the first aspect of the present invention is a tooth cleaning composition, including a weakly acidic hypochlorite solution having an effective chloride concentration of 100 (mass ppm) or more and 2000 (mass ppm) or less and a pH of 3.0 or more and 6.5 or less.

It is preferable that the tooth cleaning composition of the present invention contains a composition in a liquid, gel, or paste state containing the weakly acidic hypochlorite solution. It is more preferable that the tooth cleaning composition further contains a viscosity modifier and has a "hanging value" of 5 mm or less, in a case where the tooth cleaning composition is in a gel or paste state. In this regard, the expression "hanging value" means a value measured as follows. First, 0.1 g of the tooth cleaning composition is put within a circular area having a diameter of around 5 mm on a horizontally-placed slide glass, and left to stand for 30 seconds. After that, the slide glass is erected vertically and held for 30 seconds to allow the tooth cleaning composition to hang down. A distance (mm) from a position that is the lowest end when the slide glass is erected vertically, which is a contact portion between the tooth cleaning composition and the slide glass immediately before erecting the slide glass vertically, to the lowest end point of a contact portion after hanging the tooth cleaning composition down for 30 seconds is taken as the "hanging value".

The second aspect of the present invention is a tooth cleaning agent including the tooth cleaning composition of the first aspect of the present invention.

The third aspect of the present invention is a dental calculus removal accelerator for scaling (SC) and/or root planing (RP) including the tooth cleaning composition of the first aspect of the present invention.

The fourth aspect of the present invention is a tooth cleaning method using the tooth cleaning composition of the present invention.

### Advantageous Effects of Invention

As indicated in Examples to be described later, the tooth cleaning composition of the present invention exerts an effect of facilitating the removal of dental calculi with a scaler only by bringing the tooth cleaning composition into contact with a dental calculus-fixed tooth for an extremely short period of time of around 30 seconds. Accordingly, the burdens on a patient, a dentist, a dental hygienist, and the like can be reduced in SRP or the like in a dental clinic. Moreover, the main component of the tooth cleaning composition is a widely available weakly acidic hypochlorite solution, and the tooth cleaning composition does not require much labor for preparation and has a great advantage in terms of cost. Further, since the tooth cleaning composition contains hypochlorous acid in a molecular state, which has a high bactericidal effect, the composition also exerts bactericidal and sterilizing effects. For this reason, the tooth cleaning composition is also useful as a tooth cleaning agent for oral care performed on a daily basis in a general household.

The present inventors presume the reason why such excellent effects can be exerted as follows. That is, since the hypochlorous acid in a molecular state is an electrically neutral molecule, the hypochlorous acid has high permeability and also has strong oxidizing power, and therefore, it is considered that the hypochlorous acid decomposes an organic component contained in dental calculi, in particular, an organic component that is involved in the adhesion between the dental calculi existing near a tooth surface and the tooth surface. Further, it is considered that part of hypochlorous acid is decomposed due to the contact with the tooth surface, the surface of dental calculi, or the like, and generates hydrochloric acid, and the hydrochloric acid acts to embrittle an inorganic component of dental calculi. In addition, it is presumed that with the simultaneous occurrence of the decomposition of organic components and the embrittlement of inorganic components, the dental calculi can be easily removed.

Further, it can be deemed that as disclosed in Non Patent Literature 1, it is difficult to predict that the hypochlorous acid in a molecular state exerts such a unique effect, from the fact that a noticeable effect has not been observed in a commercially available dental calculus removal accelerator having a similar hypochlorite or the like as an active component (the active component is considered to be a chloramine derived from a hypochlorous acid ion represented by the chemical formula $OCl^-$, not the hypochlorous acid in a molecular state).

**Description of Embodiments**

The tooth cleaning composition of the present invention is characterized by containing a weakly acidic hypochlorite solution having an effective chloride concentration of 100 (mass ppm) or more and 2000 (mass ppm) or less and a pH of 3.0 or more and 6.5 or less.

In this regard, the tooth cleaning composition means a composition in which a drug is mixed, used for cleaning a natural and/or artificial tooth (including a natural tooth, a tooth restored with a dental composite resin or a dental prosthesis, dentures, and a partial denture). The tooth cleaning composition includes a dental calculus removal accelerator, a denture cleanser, a dentifrice, a so-called mouthwash, and the like. In this regard, in a case where the tooth cleaning composition of the present invention is applied to a dental prosthesis, it is preferable that the tooth cleaning composition is used for a prosthesis made of a material that hardly causes a problem of corrosion, that is, specifically, a resin, a composite resin, or a ceramic such as zirconia, or a prosthesis made of a composite material of the resin, composite resin, or ceramic.

Further, the hypochlorite solution means an aqueous solution in which hypochlorous acid in a molecular state is dissolved. The hypochlorous acid is a compound represented by the chemical formula $HClO$, and the existence form changes depending on the pH in an aqueous solution. Specifically, most of the compounds are present as molecule-type hypochlorous acid ($HClO$) in a weakly acidic region of pH around 3 to 6, the presence as the dissociated hypochlorous acid ion ($OCl^-$) predominates in a basic region of pH 9 or more, and the generation of chlorine molecules ($Cl_2$) becomes predominant as the pH decreases in a strongly acidic region (for example, pH of less than 3). In the composition for dental cleaning agent of the present invention, a weakly acidic hypochlorite solution having a pH of 3.0 or more and 6.5 or less, in which the compounds of hypochlorous acid are dissolved in the form of molecule-type hypochlorous acid ($HClO$), is used.

In this regard, among these existence forms, the molecule-type hypochlorous acid ($HClO$) has an extremely high bactericidal effect, and it is deemed that the bactericidal effect is around 80 times that of the ion-type hypochlorous acid ($OCl^-$). Since a weakly acidic hypochlorite solution, which contains molecule-type hypochlorous acid having such a high bactericidal effect in a large amount, has relatively high safety to the human body, the solution is used as a sterilizing or bactericidal agent in various fields of medical care, dentistry, agriculture, food processing, and the like. Further, in recent years, the solution has come to be used for the application of sterilization or disinfection in a public facility such as a nursing care facility, an educational facility, or a commercial facility, or in a general household, and the consumption is increasing year by year.

The pH of the weakly acidic hypochlorite solution to be used in the tooth cleaning composition of the present invention may be 3.0 or more and 6.5 or less. It is known that the self-decomposition rate of molecule-type hypochlorous acid in an aqueous solution changes depending on the pH, and it is also known that the self-decomposition rate is the highest at around pH 6.7, decreases as the pH decreases, and hardly changes at pH 5.5 or less. Therefore, by setting the pH to 6.0 or less, particularly 5.5 or less, a stable weakly acidic hypochlorous acid water can be obtained. For such a reason, it is preferable that the pH of the solution is 3.0 or more and 6.0 or less, and particularly 4.0 or more and 5.5 or less.

The effective chloride concentration in the weakly acidic hypochlorite solution to be used in the tooth cleaning composition of the present invention is 100 (mass ppm) or more and 2000 (mass ppm) or less, and preferably 300 (mass ppm) or more and 2000 (mass ppm) or less. Herein, the effective chloride concentration means the total concentration expressed in terms of chlorine of chlorine molecules dissolved in the solution, chlorine compounds having oxidizing power (for example, molecule-type hypochlorous acid), and chlorine atom-containing ions having oxidizing power (for example, ion-type hypochlorous acid). More specifically, the effective chloride concentration means the concentration obtained by converting the concentration on a mass basis of each of the components to the chlorine concentration on a mass basis, and then summing up the obtained concentrations. In a weakly acidic aqueous solution of hypochlorous acid metal salt having a pH of 3.0 or more and 6.5 or less, since almost all the hypochlorous acid are present in a molecular state, the effective chloride concentration means substantially the effective chloride concentration derived from the molecule-type hypochlorous acid.

In the present invention, as the effective chloride concentration, the concentration measured by absorptiometry (hereinafter referred to as "iodometry") with an iodine reagent is adopted. The iodometry is an analytical method that utilizes an oxidation-reduction reaction between molecule-type hypochlorous acid and iodide ions, and utilizes the reaction of the following formula.

$$KI \rightarrow K^+ + I^-$$

$$2I^- + HOCl + H^+ \rightarrow I_2 + H_2O + Cl^-$$

$$I_2 + I^- \rightarrow I_3^-$$

In the iodometry, by adding an adequate amount of acid to an object to be measured so as to make the object acidic of pH 3.0 to 6.5, and then adding potassium iodide to the acidic object, one molecule of triiodide ion showing brown color can be obtained per molecule of the molecule-type hypochlorous acid. Since the amount of triiodide ions and the amount of molecule-type hypochlorous acid in a sample are theoretically the same, the concentration of the triiodide molecule obtained by absorptiometry is the effective chloride concentration.

The measurement of effective chloride concentration by iodometry can be performed by using a commercially available effective chloride concentration measurement device, for example, an effective chloride concentration measurement kit, AQ-202 Type (SIBATA SCIENTIFIC TECHNOLOGY LTD.). Since the range of the effective chloride concentration that can be measured by an effective chloride concentration measurement kit, AQ-202 Type is 0 to 300 ppm, in a case where the effective chloride concentration of a weakly acidic hypochlorite solution to be used is higher than the above range, the weakly acidic hypochlorite solution is appropriately diluted with ion exchanged water for measurement, and then the measured value may be corrected by the dilution ratio to determine the effective chloride concentration before the dilution. In this regard, as the factor that lowers the accuracy of the measurement of effective chloride concentration by iodometry, there is volatilization of the iodine molecule generated by the above second formula. In order to increase the measurement accuracy, it is preferable to rapidly perform from the addition of potassium iodide to the measurement of triiodide molecules by absorptiometry at the time of the measurement.

The weakly acidic hypochlorite solution can be easily obtained by, for example, treating an aqueous solution of hypochlorous acid metal salt such as a sodium hypochlorite aqueous solution having a desired concentration with a weak acid ion-exchange resin. The weakly acidic hypochlorite solution may be adjusted to have a desired concentration by obtaining a hypochlorite solution in a high concentration and then diluting the solution with water.

The effective chloride concentration in the weakly acidic hypochlorite solution to be used in the tooth cleaning composition of the present invention may be 100 (mass ppm) or more and 2000 (mass ppm) or less, and preferably 300 (mass ppm) or more and 2000 (mass ppm) or less, and may also be used by adjusting appropriately the concentration depending on the use form. That is, the higher the effective chloride concentration is, the better the dental calculus removal is, but in a case where the concentration is high, the odor cannot be ignored. Therefore, in a case where such a solution is used outside the oral cavity for a denture or the like detached, it is preferable to use a solution having a high effective chloride concentration, specifically, a solution having an effective chloride concentration of 300 (mass ppm) or more and 2000 (mass ppm) or less, and particularly 500 (mass ppm) or more and 1200 (mass ppm) or less. On the other hand, in a case where such a solution is used for a natural tooth or a restored tooth, the solution is required to be applied in the human oral cavity, and in such a case, it is preferable to use a solution having an effective chloride concentration of 100 (mass ppm) or more and 1000 (mass ppm) or less, and particularly 300 (mass ppm) or more and 800 (mass ppm) or less from the viewpoint of the safety against odor and accidental ingestion or the like.

The form of the tooth cleaning composition of the present invention is not particularly limited as long as the composition contains the weakly acidic hypochlorite solution, and may be any of a liquid state, a gel state, or a paste state. In a case of a liquid state, the weakly acidic hypochlorite solution may be used as it is, or may be used with the addition of various kinds of additive agents that are soluble in water or capable of being uniformly dispersed, as needed within a range not inhibiting the effects of the present invention. Examples of the various kinds of additive agents include a water-soluble solvent, a pH adjusting agent, a coloring agent, an aromatic substance, and a sweetener. As the pH adjusting agent, a water-soluble base such as sodium hypochlorite, or sodium hydroxide, or an inorganic acid such as hydrochloric acid, nitric acid, or phosphoric acid can be suitably used.

Further, in a case of obtaining a gel state or a paste state, a viscosity modifier may be mixed with the above liquid composition. As the viscosity modifier, for example, organic particles or inorganic particles can be used. From the viewpoint of the storage stability and the operability, it is preferable to use an inorganic powder having an average primary particle diameter of 5 nm or more and 100 nm or less. With the addition of the inorganic powder in an amount of 3 to 20% by mass and particularly 6 to 15% by mass on a mass basis of the tooth cleaning composition, the composition can be made into a gel form, which is excellent in the dental calculus removal acceleration and the operability. In this regard, the average primary particle diameter (d) of the inorganic powder means, specifically, a value calculated by the following mathematical formula (1) from the specific surface area and density of the inorganic powder.

$$d = 6 / (S \times D) \quad ... \quad \text{mathematical formula (1)}$$

(In the mathematical formula (1), d represents an average primary particle diameter (pm), S represents a specific surface area ($m^2/g$), and D represents a density ($g/cm^3$) of an inorganic powder. The specific surface area is measured by using a BET-type specific surface area meter, and the density is measured by using a He-gas pycnometer method.)

As the material for the above inorganic powder, quartz, silica, titania, or the like, which is insoluble in water, is suitably used. In this regard, since a metal salt may be ionized in water and accelerate the decomposition of the hypochlorous acid in a molecular state, the metal salt is required to be carefully used. Fumed silica can be particularly suitably used because the storage stability is high, and the thickening effect is also high, which is attributed to the fact that the specific surface area is as large as 50 to 500 $m^2/g$, when the fumed silica is added. Fumed silica is one kind of fine silicas, and has a feature that is easy to obtain a high purity of 99% or more, because the fumed silica is produced by a dry method in which silicon tetrachloride is burned in an oxyhydrogen flame.

The method for adding the additive agent or the viscosity modifier is not particularly limited, and respective components may be added appropriately into the weakly acidic hypochlorite solution, the mixture may be stirred or kneaded by using a stirring device or the like, and then prepared into a dosage form that can be used as it is. Further, for example, the liquid component and the powder component may be prepared separately and packaged separately, and appropriately mixed by a practitioner such as a doctor at the time of use.

In this regard, in a case where the tooth cleaning composition of the present invention (component containing the weakly acidic hypochlorite solution in a case of being packaged separately) is stored in a container, it is preferable to store the composition in a resin or glass container from the viewpoint of suppressing the decomposition reaction of hypochlorous acid and improving the storage stability. Examples of the preferable resin include polyethylene, polypropylene, and polyethylene terephthalate, which are excellent in the oxidation resistance.

The tooth cleaning composition of the present invention functions as a tooth cleaning agent that makes the dental calculi or dental plaque in a state easier to remove by being brought into contact for a certain period of time or more with a surface of a natural tooth, a tooth restored with a dental composite resin or a dental prosthesis, or a tooth of dentures or a partial denture, and exerts an effect of cleaning the surface. At this time, the method for bringing the tooth cleaning composition of the present invention into contact with a surface of a tooth is not particularly limited as long as it is a method in which the contact can be secured for a certain period of time (for example, around 30 seconds). For example, in a case where the contact (treatment) can be conducted outside the oral cavity on a denture or the like detached, a method (a) in which an object to be treated such as a denture is immersed in the tooth cleaning composition of the present invention in a liquid state; a method (b) in which clean gauze, absorbent cotton, or the like is soaked with the tooth cleaning composition of the present invention in a liquid state, and then used to cover a surface of an object to be treated so as to be in close contact with the surface; a method (c) in which the tooth cleaning composition of the present invention in a gel or paste state is put, stuck, or applied on a surface of an object to be treated; or the like can be adopted. Further, in a case where such a composition is applied in the oral cavity to a tooth or the like that cannot be detached, in addition to the above methods (b) and (c), a method (d) in which the tooth cleaning composition of the present invention in a liquid state is continuously supplied (so-called pouring) onto a surface of an object to be treated (in this case, it is preferable to use a discharge device such as a vacuum device to constantly remove excess composition); a method (e) in which the tooth cleaning composition of the present invention in a liquid state is put into the mouth, and the mouth is rinsed with the composition; a method (f) in which the tooth cleaning composition of the present invention in a liquid state is put on a toothbrush, and brushing with the toothbrush is performed in a similar manner as in toothpaste; or the like can be adopted.

Among these methods, in the method (c), the amount of the weakly acidic hypochlorite solution to be used is smaller as compared with those in the methods (a), (d), and (e), the reliable contact can be conducted, and further the operation is simple and easy and the burden on a person to be treated (such as a patient) is small. In addition, with the injection of the tooth cleaning composition into a periodontal pocket by using a syringe, the tooth cleaning composition becomes easy to act also on the dental calculi inside the periodontal pocket. For such reasons, the method (c) can be deemed to be an excellent method.

The time for which the tooth cleaning composition of the present invention is in contact with a surface of a tooth is not particularly limited. In particular, in a case where the contact (treatment) can be conducted outside the oral cavity on a denture or the like detached, the time is preferably 10 seconds or more, and more preferably 30 seconds or more. The upper limit of the contact time is not particularly limited, and may be appropriately adjusted in consideration of the economy and efficiency. For example, in a case where the contact is conducted outside the oral cavity as described above, it is efficient to start the contact (treatment) when an object to be cleaned such as a denture is detached from the oral cavity before going to bed, and to continue the contact (treatment) until waking up, which saves time.

In a case where such a composition is applied in the oral cavity to a tooth or the like that cannot be detached, the time is preferably 10 to 300 seconds, more preferably 20 to 200 seconds, and particularly preferably 30 to 100 seconds. In a case where the time exceeds 300 seconds, the burden on the patient or the like is increased.

In the tooth cleaning method according to the present invention, it is preferable that the tooth cleaning composition is brought into contact with an object to be treated, and then the scaling and/or root planing is performed.

In adopting the above method (c), it is preferable to adjust the kind and amount of the viscosity modifier so that the tooth cleaning composition has an adequate built-up property. In this way, the tooth cleaning composition can be permeated into a narrow gap, and further the tooth cleaning composition can be retained on a surface of a tooth. Specifically, it is preferable to set the above-described "hanging value" of the tooth cleaning composition to 5 mm or less, and particularly 0.5 mm or more and 4.0 mm or less.

## Examples

Hereinafter, the present invention will be specifically described by referring to Examples and Comparative Examples, but the present invention is not limited at all by such Examples.

(Example 1)

<Preparation of weakly acidic hypochlorite solution>

A 12% by mass NaClO aqueous solution (NEOLUX Super, supplier: Shimada Shoten Co., Ltd) was diluted with ion exchanged water having an ion conductivity of 3 (mS/m) or less so that the ClO⁻ was 11400 ppm, to prepare a raw material aqueous solution. Next, 100 mL of a weak acid ion-exchange resin (Amberlite IRC-76, manufactured by ORGANO CORPORATION) was weighed out, 1200 mL of the raw material aqueous solution was added into the weighed-out weak acid ion-exchange resin, and the mixture was stirred at 23°C for 30 minutes by using a fluororesin stirring blade so that the weak acid ion-exchange resin was uniformly dispersed. During the stirring, the pH of the mixture was monitored, and when the pH decreased to reach 5.5, the stirring was terminated, and the stirring time was taken as the mixing time. In this regard, the pH was measured by using a pH meter, F-55 type (manufactured by HORIBA Ltd.)

After completion of stirring, the mixture was left to stand until the resin settled, and by decantation, a hypochlorite solution being a supernatant was recovered in a polyethylene container through a # 200 filter cloth so as not to include the resin. After the recovering, the solution was left to stand at room temperature for 4 hours in order to stabilize the pH, and pH 4.5 was obtained.

When the effective chloride concentration of the obtained weakly acidic hypochlorite solution was measured by using an effective chloride concentration measurement kit, AQ-202 Type (SIBATA SCIENTIFIC TECHNOLOGY LTD.), 11400 mass ppm was obtained.

<Preparation of tooth cleaning composition in liquid state>

The hypochlorite solution was diluted with ion exchanged water to prepare a tooth cleaning composition in a liquid state having a pH of 5.8 and an effective chloride concentration of 306 mass ppm.

By using the obtained tooth cleaning composition as a tooth cleaning agent (dental calculus removal accelerator) L1, the removal test of dental calculi was performed as follows.

<Removal test of dental calculi>

An extracted human tooth having dental calculi was washed with water, and then the water was wiped off the tooth with a Kimwipe, and the tooth was immersed in a dental calculus removal accelerator in a liquid form for 30 seconds. The extracted human tooth having dental calculi after the immersion was taken out from the dental calculus removal accelerator, and then the moisture was wiped off the tooth with a Kimwipe. The dental calculus-adhered part was scaled back and forth once with a probe (scaler), and the easiness of the removal of dental calculi was evaluated on the basis of the following evaluation criteria.

☉: Removal is particularly excellent. The dental calculi adhered are removed as a lump by one scaling.

○: Removal is excellent. The dental calculi adhered are removed by one scaling.

Δ: Immersion is required multiple times for the removal of dental calculi.

×: Dental calculi cannot be removed even after performing the immersion and scaling 10 times or more.

When a test was conducted on each of five extracted human teeth by using a dental calculus removal accelerator L1, the evaluation results were "○" for all of the five teeth.

(Examples 2 to 6)

Dental calculus removal accelerators each in a liquid state, which have the effective chloride concentrations and pHs shown in Table 1, respectively, were prepared in a similar manner as in Example 1 except that the dilution ratio of the weakly acidic hypochlorite solution was changed, and the evaluations were made in a similar manner as in Example 1. The results are shown in Table 1.

(Comparative Example 1)

The evaluation was made in a similar manner as in Example 1 except that ion exchanged water was used in place of the dental calculus removal accelerator L1. The results are shown in Table 1.

(Comparative Example 2)

A 12% sodium hypochlorite (NaClO) aqueous solution (NEOLUX Super, supplier: Shimada Shoten Co., Ltd) was diluted around 150 times (on a mass basis) with ion exchanged water to prepare a NaClO aqueous solution having an effective chloride concentration (substantially ClO⁻ concentration) of 840 mass ppm and a pH of 9.4. The evaluation was made in a similar manner as in Example 1 except that the aqueous solution was used in place of the dental calculus removal accelerator L1 as the dental calculus removal accelerator. The results are shown in Table 1.

[Table 1]

| No. | | Dental calculus removal accelerator | Effective chloride concentration (mass ppm) | pH | Dental calculus removal |
|---|---|---|---|---|---|
| Example | 1 | L1 | 306 | 5.8 | ○ 5 teeth |
| | 2 | L2 | 510 | 5.7 | ☉ 2 teeth<br>○ 3 teeth |
| | 3 | L3 | 690 | 5.6 | ☉ 2 teeth<br>○ 3 teeth |
| | 4 | L4 | 840 | 5.5 | ☉ 4 teeth<br>○ 1 tooth |
| | 5 | L5 | 1020 | 5.5 | ☉ 5 teeth |
| | 6 | L6 | 1200 | 5.4 | ☉ 5 teeth |
| Comparative Example | 1 | Ion exchanged water | 0 | 6.2 | × 2 teeth |
| | 2 | NaClO aqueous solution | 840 | 9.4 | Δ 5 teeth |

(Example 7)

A hypochlorite solution having an effective chloride concentration of 840 mass ppm was obtained in a similar manner as in Example 4, 3% by mass of QS-102 (manufactured by Tokuyama Corporation, fumed silica having an average primary particle diameter of 14 nm, a BET specific surface area of 200 $m^2/g$, and a density of 2.2 $g/cm^3$) was added into the hypochlorite solution as the inorganic powder, and the mixture was stirred for one hour with a stirrer to prepare a dental calculus removal accelerator in a gel form.

The obtained dental calculus removal accelerator in a gel form was evaluated for the ease of extrusion from a syringe, the built-up property (hanging value), the consistency (easiness of deformation of gel form), and the dental calculus removal, as follows.

<Evaluation of ease of extrusion from syringe>

Terumo Syringe SS-01ES was filled with 1 mL of a dental calculus removal accelerator, a Tokuyama Dispensing Tip (manufactured by Tokuyama Dental Corporation) was attached to the Syringe, and then the whole amount was extruded. At that time, the case where the whole amount was extruded without resistance was marked as good (○), and the case where resistance was felt in the syringe was marked as poor (×).

<Evaluation of built-up property>

0.1 g of a dental calculus removal accelerator was put on a horizontally-placed slide glass so that the contact portion in a state of being left to stand had a circular shape (around 20 mm$^2$) having a diameter of around 5 mm, and was left to stand for 30 seconds, and then the slide glass was erected vertically and held for 30 seconds to allow the tooth cleaning composition to hang down. A distance (mm) from the lowest end point of the contact portion in a state of being left to stand to the lowest end point of the contact portion after the hanging down for 30 seconds was taken as the "hanging value". When the "hanging value" was 5 mm or less, the built-up property was evaluated as good (○), and when the "hanging value" exceeded 5 mm, the built-up property was evaluated as poor (×).

<Evaluation of consistency>

0.1 g of a dental calculus removal accelerator was weighed onto a square-shaped polypropylene sheet of 5 cm square, and covered with another polypropylene sheet having the same size so as not to be weighted. Next, a plate-shaped weight having a horizontal surface of 6 cm square was placed so as to weigh 50 g together with the mass of the polypropylene sheet covering the dental calculus removal accelerator. After weighting for 10 seconds, the weight was lifted to release the weight. The major axis X (mm) of the dental calculus removal accelerator spread in a substantially circular shape between the polypropylene sheets, and the diameter Y (mm) of the dental calculus removal accelerator perpendicular to the major axis were measured, and the average value (mm) of X and Y was taken as the consistency. When the consistency exceeds 25 mm, the dental calculus removal accelerator is in a loose gel form, is easily deformed, and easily hangs down, and when the consistency is 15 mm or less, the dental calculus removal accelerator is in a hard gel form, and the feeling of extrusion from a syringe becomes heavy.

<Evaluation of dental calculus removal>

An extracted human tooth having dental calculi was washed with water, and then the water was wiped off the tooth with a Kimwipe. The dental calculus-adhered part was coated with the dental calculus removal accelerator, and the coated part was left to stand for 30 seconds. The dental calculus removal accelerator was wiped off the dental calculus-adhered part with a Kimwipe, and then the part was scaled back and forth once with a probe, and the easiness of the removal of dental calculi was evaluated. The evaluation criteria for the dental calculus removal are the similar to those described in Example 1.

[Table 2]

| | | Dental calculus removal accelerator | Effective chloride concentration (mass ppm) | pH | QS-102 (mass%) | Dental calculus removal | Built-up property: hanging value (mm) | Ease of extrusion | Consistency (mm) |
|---|---|---|---|---|---|---|---|---|---|
| Example | 7 | G1 | 840 | 5.5 | 3 | ⊙ 4 teeth<br>○ 1 tooth | ×: 5.5 | ○ | 27.1 |
| | 8 | G2 | 840 | 5.5 | 6 | ⊙ 4 teeth<br>○ 1 tooth | ○: 4.5 | ○ | 21.4 |
| | 9 | G3 | 840 | 5.5 | 11 | ⊙ 4 teeth<br>○ 1 tooth | ○: 1.3 | ○ | 19.5 |
| | 10 | G4 | 840 | 5.5 | 15 | ⊙ 4 teeth<br>○ 1 tooth | ○: 0.5 | ○ | 17.6 |
| | 11 | G5 | 840 | 5.5 | 20 | ⊙ 4 teeth<br>○ 1 tooth | ○: 0.0 | × | 15 |

In the dental calculus removal accelerators of Examples, each of which contains hypochlorous acid in a molecular state, favorable dental calculus removal was obtained in either the liquid state or the gel state by the contact (treatment) with a tooth (dental calculi) for 30 seconds. In contrast, in Comparative Example 1 in which ion exchanged water was used, the dental calculus removal was not improved at all. Further, in Comparative Example 2 in which hypochlorous acid ions were used, the dental calculus removal was evaluated as $\Delta$, which is inferior to those of Examples.

**Claims**

1. A tooth cleaning composition, comprising a weakly acidic hypochlorite solution having an effective chloride concentration of 100 (mass ppm) or more and 2000 (mass ppm) or less and a pH of 3.0 or more and 6.5 or less.

2. The tooth cleaning composition according to claim 1,
   wherein the composition contains the weakly acidic hypochlorite solution, and the composition is in the form of liquid, gel or paste.

3. The tooth cleaning composition according to claim 2, further contains a viscosity modifier,
   wherein the composition has a "hanging value" of 5 (mm) or less, the hanging value being defined by a distance (mm) from a lowest end point of a contact portion in a state of being left to stand to a lowest end point of the contact portion after hanging down for 30 seconds when 0.1 g of the tooth cleaning composition is put within a circular area having a diameter of 5 (mm) on a horizontally-placed slide glass and left to stand for 30 seconds, and then the slide glass is erected vertically and held for 30 seconds to allow the tooth cleaning composition to hang down.

4. A tooth cleaning agent, comprising the tooth cleaning composition according to any one of claims 1 to 3.

5. A dental calculus removal accelerator for scaling (SC) and/or root planing (RP), comprising the tooth cleaning composition according to any one of claims 1 to 3.

6. A tooth cleaning method, comprising: bringing the tooth cleaning composition according to any one of claims 1 to 3 into contact with an object to be treated, and then applying scaling and/or root planing to the object.

## INTERNATIONAL SEARCH REPORT

| | International application No. |
|---|---|
| | PCT/JP2019/034516 |

### A. CLASSIFICATION OF SUBJECT MATTER

Int.Cl. A61K8/20(2006.01)i, A61K8/02(2006.01)i, A61K9/06(2006.01)i, A61K9/08(2006.01)i, A61K33/20(2006.01)i, A61P1/02(2006.01)i, A61Q11/00(2006.01)i

According to International Patent Classification (IPC) or to both national classification and IPC

### B. FIELDS SEARCHED

Minimum documentation searched (classification system followed by classification symbols)

Int.Cl. A61K8/20, A61K8/02, A61K9/06, A61K9/08, A61K33/20, A61P1/02, A61Q11/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>Y | WO 2007/072697 A1 (NOGUCHI DENTAL MEDICAL RESEARCH INSTITUTE) 28 June 2007, claim 1, paragraph [0127], examples 1, 5 (Family: none) | 1-2,4-6<br>3-6 |
| X<br>Y | JP 2008-260740 A (ASAHI PRETEC CORP.) 30 October 2008, claim 1, paragraphs [0013], [0015]-[0017], [0026], [0028], examples 1, 2, 5 (Family: none) | 1-4<br>3-6 |

☒ Further documents are listed in the continuation of Box C. ☐ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 08 November 2019 (08.11.2019) | 19 November 2019 (19.11.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2019/034516

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 3-279330 A (KURARAY CO., LTD.) 10 December 1991, page 2, lower right column, line 14 to page 3, upper left column, line 3 (Family: none) | 3-6 |
| Y | 西恭宏，基礎から学ぼう口腔ケア 義歯編(5) 義歯の手入れ，月刊総合ケア, vol. 13, no. 8, 15 August 2003, pp.86-87, non-official translation (NISHI, Yasuhiro, "Learn oral care from the basics, Denture edition (5), Caring for dentures", The Japanese journal of total care) | 5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H09143043 A
- JP 4588348 B
- JP 2007126409 A

### Non-patent literature cited in the description

- **M. BECKER et al.** *International Journal of Dental Hygiene,* 2018, vol. 16, 151-156